# EUROPEAN PATENT SPECIFICATION

(11) **EP 4 538 300 B1**
(45) Date of publication and mention of the grant of the patent: **26.11.2025**
(21) Application number: 23203437.1
(22) Date of filing: 13.10.2023
(51) Int. Cl.: C08B 37/02, A61K 47/36

(54) **ONE POT GMP SCALABLE PRODUCTION METHOD FOR THE SYNTHESIS OF DEXTRAN-GUANIDINE-BISPHOSPHONATE CONJUGATE**
SKALIERBARES EINTOPF-GMP-HERSTELLUNGSVERFAHREN ZUR SYNTHESE VON DEXTRAN-GUANIDIN-BISPHOSPHONAT-KONJUGAT
PROCÉDÉ DE PRODUCTION ÉVOLUTIVE DE GMP EN UN SEUL RÉCIPIENT POUR LA SYNTHÈSE D'UN CONJUGUÉ DEXTRANE-GUANIDINE-BISPHOSPHONATE

(43) Date of publication of application: 16.04.2025
(73) Proprietor: Dextech Medical AB, 75106 Uppsala (SE)
(72) Inventor: HOLMBERG, Anders R, 75106 Uppsala (SE)
(74) Representative: Boco IP Oy Ab

(56) References cited:
- EP-B1- 2 274 018
- HOLMBERG: "Development of a novel poly bisphosphonate conjugate for treatment of skeletal metastasis and osteoporosis", INTERNATIONAL JOURNAL OF ONCOLOGY, vol. 37, no. 3, 21 July 2010 (2010-07-21), GR, XP093137302, ISSN: 1019-6439, DOI: 10.3892/ijo_00000705
- MARCELA MARQUEZ ET AL: "Charge-dependent Targeting: Results in Six Tumor Cell Lines", ANTICANCER RESEARCH, 24 February 2004 (2004-02-24), pages 1 - 5, XP093222908, Retrieved from the Internet <URL:https://ar.iiarjournals.org/content/24/3A/1347.short>

## Description

### FIELD OF THE DISCLOSURE

The present disclosure relates to the synthesis of a modified dextran conjugate, and more particularly to a large scale GMP synthesis method resulting in a pharmaceutical quality dextran-guanidine-bisphosphonate conjugate product. The present disclosure further concerns the use of said dextran-guanidine-bisphosphonate conjugate in medicines treating tumors.

### BACKGROUND OF THE DISCLOSURE

Dextran-guanidine-bisphosphonate conjugate is a drug candidate for treatment of cancer metastases located in the bone and associated with increased bone cell activity. The dextran-guanidine-bisphosphonate conjugate has been investigated in clinical studies (phase 1 and phase 2) and it has a beneficial safety profile with treatment efficacy and with no drug-related serious adverse events recorded (Thellenberg-Karlsson et al. 2023).

Holmberg et al. 2010 and Holmberg et al. 2009 disclose biological effects of the dextran-guanidine-bisphosphonate conjugate. The present inventor's patent publications EP2131867B1 and EP2274018B1 describe the function and properties of dextran-guanidine-bisphosphonate conjugates, as well as micro scale method for preparation of said conjugates. The synthesis methods employed cannot be translated to clinical research but in general requires extensive process development. Most often the translation fails, either that positive results cannot be replicated in human subjects or that the study drug cannot be manufactured at large GMP standard scale. Unforeseen toxicity may also be a reason for failure.

The translation of drug development from pre-clinical research to clinical research has several strict regulatory requirements especially regarding the manufacture of the drug candidate. It requires a larger-scale GMP synthesis production method that results in a pharmaceutical grade product.

The method employed must be approved in every detail by the medical authority of the country (countries) where the clinical research is planned to take place. Usually, the requirements increase when the drug candidate advances in the clinical research phases.

In Europe, The European Medicines Agency (EMA), stipulates the general requirements for good manufacturing practice (GMP). GMP describes "the minimum standard that a medicines manufacturer must meet in their production processes. EMA coordinates inspections to verify compliance with these standards and plays a key role in harmonising **GMP** activities at EU level". Further, GMP requires that medicines are of consistent high quality and are appropriate for their intended use.

The GMP requirements are employed in every detail of the synthesis described in the investigational medicinal dossier (IMPD).

Clinical research of the dextran-guanidine-bisphosphonate conjugate requires a large-scale synthesis producing synthesis resulting in a pharmaceutical grade product.

### BRIEF DESCRIPTION OF THE DISCLOSURE

An object of the present disclosure is to provide a large-scale GMP synthesis that is also an optimized improved method of manufacture of a dextran-guanidine-bisphosphonate conjugate, wherein the bisphosphonate group is preferably an alendronate group.

The object of the disclosure is achieved by a synthesis process which is characterized by what is stated in the independent claim.

The preferred embodiments of the disclosure are disclosed in the dependent claims.

The process is a sequential, one pot process, comprising the steps of dextran activation, alendronate coupling, aminoguanidine coupling and reductive amination, followed by purification by tangential flow filtration through optimized filtration cycles.

The present disclosure produces high purity, pharmaceutical grade (GMP) dextran-guanidine-bisphosphonate conjugates in which one molecule of dextran contains approximately five to eight molecules of alendronate and approximately 40-45 molecules of aminoguanidine. The substitution relationship results in a conjugate with affinity to remodelling bone and tumor cell cytotoxicity, a dual efficacy.

The acidic pH in the dextran activation ensures efficient activation at short reaction time. Using a buffering agent and controlling the pH of the alendronate coupling ensures that the alendronate remains soluble and prevents its precipitation. The subsequent aminoguanidine coupling as well as the reductive amination are conducted without isolation steps. Methods described in prior art are carried out in mL scale and do not allow synthesis in larger scale (L), for example because of precipitations and challenges in purification methods.

Yet another advantage of the present disclosure is that the purification process with tangential flow filtration has been optimized to ensure higher purity and higher synthesis yield of the polydispersed product. The pH of the drug product solution is adjusted to pH 6.0 - pH 7.0 to ensure complete sustained solubility of the drug product. In these conditions the drug product is stable and has an extended shelf-life.

In the present method the obtained dextran-guanidine-bisphosphonate is diluted with sodium acetate and sodium chloride, pH is adjusted to pH 6.0 - pH 7.0 and the product is filtered through bacteria retentive filters.

The present inventor found that when sodium acetate is used it prevents the guanidine and alendronate groups to react and form side-products, which would result in harmful precipitation.

The present disclosure is also related to the use of the dextran-guanidine-bisphosphonate conjugate as tumor killing treatment for cancers located in bone.

### BRIEF DESCRIPTION OF THE DRAWINGS

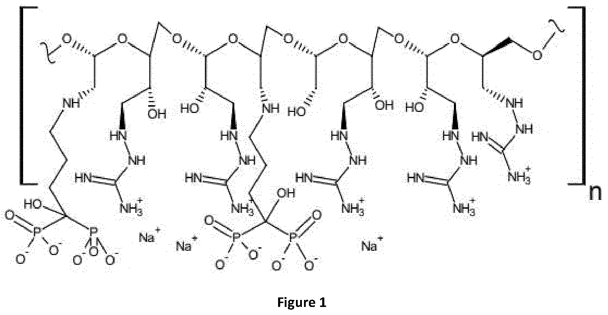

In the following the disclosure will be described in greater detail by means of preferred embodiments with reference to the accompanying drawings, in which
Figure 1 shows a principal molecular structure of the dextran-guanidine-bisphosphonate conjugate obtained with the chemistry of the present disclosure;

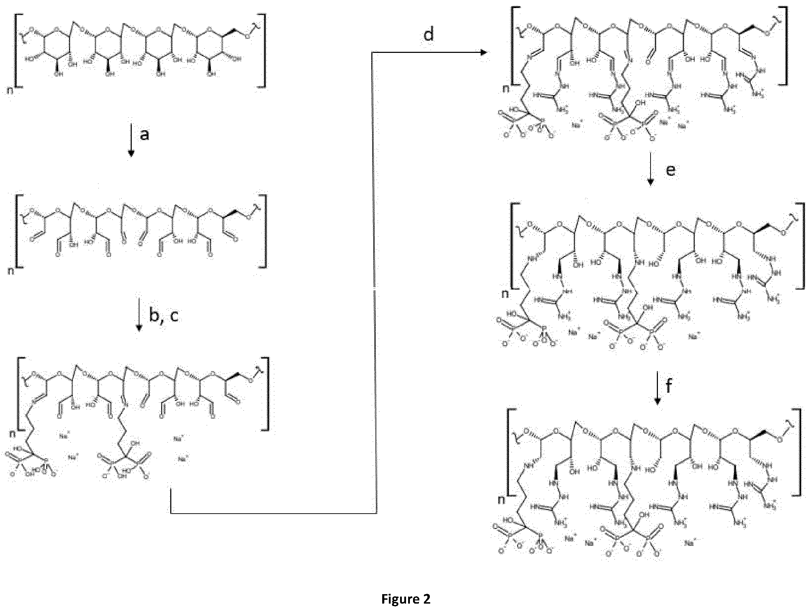

Figure 2 shows the synthesis route for the dextran-guanidine-bisphosphonate conjugate, comprising the steps of a) + b) dextran activation, c) alendronate coupling, d) aminoguanidine coupling, e) reductive amination, f) purification.

### DETAILED DESCRIPTION OF THE DISCLOSURE

The disclosure relates to a method for synthesizing a dextran-guanidine-bisphosphonate conjugate, characterized in that the method comprises the sequence of steps of:
(a) adding periodate salt to an aqueous solution comprising dextran, followed by addition of sulphuric acid,
(b) adding sodium dihydrogen phosphate to the solution obtained in step (a), followed by addition of sodium hydroxide,
(c) adding alendronic acid to the solution obtained in step (b),
(d) adding aminoguanidine to the solution obtained in step (c),
(e) adding sodium borohydride solution to the solution obtained in step (d) to produce a dextran-guanidine-bisphosphonate conjugate,
(f) purifying the dextran-guanidine-bisphosphonate conjugate obtained in step (e), preferably the purification is carried out by tangential flow filtration, to produce a purified dextran-guanidine-bisphosphonate conjugate,
(g) diluting the purified dextran-guanidine-bisphosphonate conjugate obtained in step (f) with sodium acetate and sodium chloride to produce a diluted dextran-guanidine-bisphosphonate conjugate, adjusting pH of the diluted dextran-guanidine-bisphosphonate conjugate to pH 6.0 - pH 7.0 and filtering the diluted dextran-guanidine-bisphosphonate.

According to an embodiment of the disclosure, the bisphosphonate is an aminobisphosphonate, preferably an alendronate group.

According to another embodiment of the disclosure, in step (a) pH is adjusted to pH 1.5 - pH 1.9, preferably to pH 1.55 - pH 1.8, more preferably to pH 1.6 - pH 1.7.

According to yet another embodiment, in step (b) pH is adjusted to pH 7 - pH 7.7, preferably to pH 7.3 - pH 7.7, more preferably to pH 7.3 - pH 7.5.

According to an embodiment of the disclosure, the step (a), step (c), or both step (a) and step (c), is conducted by stirring the solution in the dark.

The step (a) may be carried out for 40 to 50 minutes, preferably for 45 minutes.

The step (c) may be carried out for 55 to 70 minutes, preferably for 60 minutes.

According to an embodiment of the disclosure, the step (d), step (e), or both step (d) and step (e) is conducted by stirring the solution at room temperature.

The step (d) may be carried out for 175 to 190 minutes, preferably for 180 minutes.

The step (e) may be carried out for 17 to 20 hours, preferably for 18 hours.

In an embodiment, the synthesis consists of dextran activation in step (a), alendronate coupling in steps (b) and (c), aminoguanidine coupling in step (d), and reductive amination in step (e). Dextran activation is done in step (a), wherein an aqueous solution of dextran, preferably dextran 40, is oxidized with sodium periodate at acidic pH adjusted to pH 1.5-pH 1.9, preferably to pH 1.55 - pH 1.8, and most preferably to pH 1.6 - pH 1.7, by adding sulphuric acid to the solution until the desired pH is achieved. The oxidation is conducted by stirring the reaction mixture in the dark at room temperature for approximately 45 minutes.

The "dextran 40" means a pharmaceutical grade, low molecular dextran with an average molecular weight of 40 000 g/mol. Dextran is a linear glucose polymer, with glucose (C₆H₁₂O₆) as the repeating unit.

By "in the dark" is to be understood that the reaction is done in a room with no windows and no light on.

By "room temperature" is to be understood that the reaction is not subjected to any heating or cooling, in a room with temperature around 15-25 °C, preferably 18-22 °C, more preferably 20-22 °C.

After step (a), the pH of the solution is adjusted to pH 7 - pH 7.8, preferably to pH 7.2 - pH 7.7, more preferably to pH 7.3 - pH 7.5, with sodium dihydrogen phosphate and sodium hydroxide in step (b). Alendronate is charged into the reaction in step (c) and the reaction mixture is stirred in the dark at room temperature for approximately one hour.

After completing steps (b) and (c), aminoguanidine hydrochloride is charged to the solution in step (d) and the reaction mixture is stirred for approximately three hours at room temperature.

After completing step (d), sodium borohydride solution is added to the reaction mixture in step (e), and the reaction mixture is stirred at room temperature for 14-15 hours.

According to an embodiment, the dextran-guanidine-bisphosphonate conjugate is purified by tangential flow filtration in step (f). The material obtained after step (e) is concentrated using sodium chloride and the concentrate is diafiltrated, in which process the dextran-guanidine-bisphosphonate conjugate is purified to high purity.

According to a preferred embodiment, the material obtained after step (e) is concentrated by a factor of 3.5 and the concentrate is diafiltrated with 7 diafiltration volumes of 0.9 % sodium chloride solution over a 1 kDa, preferably 1 kDa - 5 kDa, nominal weight cutoff tangential flow filtration (TFF) membrane, in which process the dextran-guanidine-bisphosphonate conjugate is purified to high purity.

The term "tangential flow filtration (TFF)" refers to a widely used separation method in bio-pharmaceutical industry. The process, tangential flow filtration, is also known as cross-flow filtration. After tangential flow filtration the concentrated product is filtered through bacteria retentive filters. The concentrated product is sampled for the determination of active substance, based on dry weight measurement after sample lyophilization and additional drying in the oven.

According to an embodiment, in step (g) the obtained dextran-guanidine-bisphosphonate is filtered through bacteria retentive filters. According to a preferred embodiment, the product is diluted with sodium acetate and 0.9 % sodium chloride to a concentration of 22 - 28 mg/mL, more preferably 25 mg/mL ± 10 %, pH is adjusted to pH 6.0 - pH 7.0 and the product is filtered through bacteria retentive Pall Kleenpak Supor EKV 0.2 µm filters. Typically, a 0.2 µm filter is used. An example of such filter is Pall Kleenpak Supor EKV 0.2 µm filters. At this pH 6.0 - pH 7.0 the guanidine side groups are completely protonated which ensures that the drug product is soluble and stable. The drug product typically has an extended approved shelf life, such as 36 months and can thus be safely stored in 10 mL vials from which the drug product can be administered.

After the filtration the product is sampled for quality control. A skilled person in the art is aware of suitable quality control measures and can choose the method to be used.

In a preferred embodiment, the present disclosure relates to a method for synthesizing a dextran-guanidine-bisphosphonate conjugate, wherein the method comprises the sequence of steps of:
(a) adding periodate salt to an aqueous solution comprising dextran, followed by addition of sulphuric acid, wherein pH is adjusted to pH 1.5 - pH 1.9, preferably to pH 1.55 - pH 1.8, more preferably to pH 1.6 - pH 1.7, and stirring the solution in the dark at room temperature for approximately 45 minutes,
(b) adding sodium dihydrogen phosphate to the solution obtained in step (a), followed by addition of sodium hydroxide, wherein pH is adjusted to pH 7 - pH 7.8, preferably to pH 7.2 - pH 7.7, more preferably to pH 7.3 - pH 7.5.
(c) adding alendronic acid to the solution obtained in step (b), and stirring the solution in the dark at room temperature for approximately one hour,
(d) adding aminoguanidine to the solution obtained in step (c), and stirring the solution at room temperature for approximately three hours,
(e) adding sodium borohydride solution to the solution obtained in step (d), and stirring the solution at room temperature for 14 to 15 hours, to produce a dextran-guanidine-bisphosphonate conjugate.
(f) purifying the dextran-guanidine-bisphosphonate conjugate obtained in step (e), preferably the purification is carried out by tangential flow filtration, to produce a purified dextran-guanidine-bisphosphonate conjugate,
(g) diluting the purified dextran-guanidine-bisphosphonate conjugate obtained in step (f) with sodium acetate and sodium chloride to produce a diluted dextran-guanidine-bisphosphonate conjugate, adjusting pH of the diluted dextran-guanidine-bisphosphonate conjugate to pH 6.0 - pH 7.0 and filtering the diluted dextran-guanidine-bisphosphonate.

In step (g) the dextran-guanidine-bisphosphonate conjugate is diluted with sodium acetate and sodium chloride to a concentration of 22 - 28 mg/mL, preferably to 25 mg/mL ± 10 %, pH is adjusted to pH 6.0 - pH 7.0 and filtered through bacteria retentive filters.

In another preferred embodiment, the present disclosure relates to a method for synthesizing a dextran-guanidine-bisphosphonate conjugate, wherein the method comprises the sequence of steps of:
(a) adding periodate salt to an aqueous solution comprising dextran, followed by addition of sulphuric acid, wherein pH is adjusted to pH 1.6 - pH 1.7, and stirring the solution in the dark at room temperature for approximately 45 minutes,
(b) adding sodium dihydrogen phosphate to the solution obtained in step (a), followed by addition of sodium hydroxide, wherein pH is adjusted to pH 7 - pH 7.8, more preferably to pH 7.3 - pH 7.5.
(c) adding alendronic acid to the solution obtained in step (b), and stirring the solution in the dark at room temperature for approximately one hour,
(d) adding aminoguanidine to the solution obtained in step (c), and stirring the solution at room temperature for approximately three hours,
(e) adding sodium borohydride solution to the solution obtained in step (d), and stirring the solution at room temperature for 14 to 15 hours, to produce a dextran-guanidine-bisphosphonate conjugate.
(f) purifying the dextran-guanidine-bisphosphonate conjugate obtained in step (e), preferably the purification is carried out by tangential flow filtration, to produce a purified dextran-guanidine-bisphosphonate conjugate,
(g) diluting the purified dextran-guanidine-bisphosphonate conjugate obtained in step (f) with sodium acetate and sodium chloride to produce a diluted dextran-guanidine-bisphosphonate conjugate, adjusting pH of the diluted dextran-guanidine-bisphosphonate conjugate to pH 6.0 - pH 7.0 and filtering the diluted dextran-guanidine-bisphosphonate.

According to a preferred embodiment, the product is diluted with sodium acetate and 0.9 % sodium chloride to a concentration of 22 - 28 mg/mL, more preferably 25 mg/mL ± 10 %, pH is adjusted to pH 6.0 - pH 7.0 and the product is filtered through bacteria retentive Pall Kleenpak Supor EKV 0.2 µm filters.

The present improved method results in a dextran-guanidine-bisphosphonate conjugate, wherein the conjugate is a dextran substituted with a guanidine group and a bisphosphonate group and has at least one free amino group. Said bisphosphonate and guanidine groups are covalently coupled to activated hydroxyl groups of said hydroxy polymer, said conjugate having tumor cell killing effect.

In an embodiment, the guanidine group is agmatine or aminoguanidine.

In an embodiment, the bisphosphonate group is an aminobisphosphonate, preferably alendronate.

Preferably, the present improved method results in a dextran-guanidine-alendronate conjugate, wherein the conjugate is a dextran substituted with a guanidine group and an alendronate group. Said guanidine group and alendronate group are covalently coupled to activated hydroxyl groups of said hydroxy polymer, said conjugate having tumor cell killing effect.

The present disclosure relates to a dextran-guanidine-bisphosphonate conjugate obtained by the method of the present disclosure.

According to an embodiment of the disclosure, one molecule of dextran-guanidine-bisphosphonate conjugate contains approximately five to eight molecules of alendronate and approximately 40-45 molecules of aminoguanidine.

One molecule of dextran-guanidine-bisphosphonate conjugate may contain five (5), six (6), seven (7), or eight (8) molecules of alendronate.

One molecule of dextran-guanidine-bisphosphonate conjugate may contain 40, 41, 42, 43, 44, or 45 molecules of aminoguanidine.

One molecule of dextran-guanidine-bisphosphonate conjugate may contain any combination of five (5), six (6), seven (7), or eight (8) molecules of alendronate, and of 40, 41, 42, 43, 44, or 45 molecules of aminoguanidine.

The disclosure also relates to a pharmaceutical formulation, wherein the pharmaceutical formulation comprises the dextran-guanidine-bisphosphate conjugate according to the present disclosure, a pharmaceutically acceptable salt or solvate thereof, and at least one pharmaceutically acceptable adjuvant.

In an embodiment, the dextran-guanidine-bisphosphonate conjugate obtained by the method presented in the present disclosure is administered to a subject in need as a solution with concentration of approximately 25 mg/mL of the conjugate in 0.1 M sodium acetate, 0.9 % NaCl (aq). Molecular weight distribution of the polydispersed drug substance is typically 0.4-95 kDa (Mw, weight average), or 0.3-90 kDa (Mn, number average).

According to an embodiment of the disclosure, the dextran-guanidine-bisphosphonate conjugate or the pharmaceutical formulation is for use in the treatment of cancer.

According to an embodiment of the disclosure, the dextran-guanidine-bisphosphonate conjugate or the pharmaceutical formulation is for use in the treatment of cancer that is a cancer located in bone.

According to an embodiment, the dextran-guanidine-bisphosphonate conjugate or the pharmaceutical formulation is for use in the treatment of cancer selected from the group of cancers metastatic to the skeleton or cancers having bone as primary site, preferably the cancer is selected from the group consisting of multiple myeloma, metastatic castration resistant prostate cancer (mCRPC), bone metastatic lung cancer, and bone metastatic breast cancer.

The dextran-guanidine-bisphosphonate conjugate of the present disclosure is preferably administered by intravenous administration. The dextran-guanidine-bisphosphonate conjugate is a sterile concentrate for solution for infusion, wherein one glass vial typically contains said conjugate in sodium acetate, and sodium chloride solution.

In an embodiment the dextran-guanidine-bisphosphonate concentrate for intravenous administration after dilution contains 10 mL of 25 mg/mL solution of the conjugate in 0.10 M sodium acetate, 0.9 % sodium chloride solution.

### EXAMPLE

### Starting materials for synthesis

Pharmaceutical grade dextran 40 was supplied by Pharmacosmos AS, Denmark. Pharmaceutical grade sodium alendronate and aminoguanidine (purity ≥ 98.0 %) were supplied by Chemrio International Limited, China.

### Raw materials for synthesis and formulation

Sodium dihydrogen phosphate monohydrate, sodium hydroxide pellets, sodium metaperiodate, sodium borohydride, hydrochloric acid fuming 37 %, sulphuric acid 95-98 %, sodium hydroxide solution 10 mol/L, sodium chloride and sodium acetate trihydrate were supplied by Merck AG, Germany.

### Synthesis

Pharmaceutical quality dextran 40 was dissolved in 6 L of water in a glass reactor by adding dextran in three batches (60 g + 60 g + 60 g, 10 g/L) and each batch was allowed to dissolve completely before adding the next batch. The solution was stirred (250 rpm) for 14-20 h at room temperature to complete the dissolving. Sodium periodate (108 g, 18 g/L) was slowly added over the surface of the clear dextran solution during stirring (500 rpm) in the dark. When sodium periodate was completely dissolved, the pH of the solution was adjusted to 1.65 ± 0.5 by adding sulphuric acid (estimated need 7 mL) in the dark. The solution was incubated under stirring (500 rpm) for 45 minutes in the dark at room temperature, after which sodium dihydrogen phosphate monohydrate (83 g, 13.8 g/L) was added. The pH was adjusted to 7.5 ± 0.2 by adding 5 M NaOH (estimated need 170 mL).

Alendronic acid was added in three batches (50 g + 50 g + 50 g, 8.33 g/L) by distributing it over the surface of the reaction mixture and each batch was allowed to dissolve before adding the next batch, after which the solution was stirred for 30 minutes to complete the dissolving. The reaction mixture was incubated under stirring (700 rpm) for 60 minutes at room temperature.

Aminoguanidine was added in three batches (240 g + 240 g + 240 g, 80 g/L) by distributing it over the surface of the reaction mixture and allowed to dissolve. The reaction mixture was incubated under stirring (700 rpm) for 180 minutes at room temperature.

Sodium borohydride solution for the next step was prepared as follows: sodium borohydride (60 g, 10 g/L) was added into 0.1 M NaOH (500 mL, 83.3 g/L) and stirred for at least 30 minutes. The mixture may be turbid. The mixture was slowly added into the reaction mixture while gently stirring (250 rpm). After complete addition and dissolving, the reaction was incubated under gentle stirring (100 rpm) for 14-20 h at room temperature, during which pH will increase approximately to pH 9.5.

### Purification

The obtained dextran-guanidine-alendronate was purified by concentrating the solution by a factor of 3.5 and diafiltrating the concentrate with 7 diafiltration volumes of 0.9 % sodium chloride solution over a 1 kDa Nominal weight cutoff tangential flow filtration (TFF) membrane.

Concentrated product was filtered through bacteria retentive Pall Kleenpak Supor EKV 0.2 µm filters and sampled for the determination of active substance based on dry weight measurement after sample lyophilization and additional drying in the oven.

The obtained dextran-guanidine-alendronate was diluted with sodium acetate and 0.9 % sodium chloride to a concentration of 25 mg/mL ± 10 %, pH is adjusted to 6.0-7.0 with 0.1 M sodium acetate, and filtered through bacteria retentive Pall Kleenpak Supor EKV 0.2 µm filters, after which it was subjected to determination of active substance, nitrogen, phosphate, free guanidine, free phosphorus, boron, iodine, content and lead content, identity, molecular weight distribution, pH, endotoxins and total aerobic microbial count (TAMC)/total yeasts and mold count (TYMC).

### REFERENCES

EP2131867B1
EP2274018B1
WO2009124580 A1
Holmberg A., Lerner U., Alayia A., Mohanna M., Adra C., Marquez M., Meurling L., Nilsson S. Development of a novel poly bisphosphonate conjugate for treatment of skeletal metastatis and osteoporosis, J. Oncolocy, 2010, Vol 36, p. 563;
Holmberg A., Marquez M., Meurling L., Nilsson S., Polymer-conjugated guanidine is a potentially useful anti-tumor agent, J. Oncology, 2009, Vol 35, p. 281.
Thellenberg-Karlsson, C., Vjaters, E., Kase, M., Tammela, T., Ojamaa, K., Norming, U., Nyman, C., Andersson, S-O., Hublarovs, O., Marquez-Holmberg, M., Castellanos, E., Ullen, A., Holmberg, A., Nilsson, S., A Randomized, double-blind, dose-finding, phase II multicentre study of ODX in the treatment of patients with castration-resistant prostate cancer and skeletal metastases, Eur. J. Cancer, 2023, 181, 198.

## Claims

1. A method for synthesizing a dextran-guanidine-bisphosphonate conjugate, **characterized in that** the method is a large scale one pot process and the method comprises the sequence of steps of:
(a) adding periodate salt to an aqueous solution comprising dextran, followed by addition of sulphuric acid,
(b) adding sodium dihydrogen phosphate to the solution obtained in step (a), followed by addition of sodium hydroxide,
(c) adding alendronic acid to the solution obtained in step (b),
(d) adding aminoguanidine to the solution obtained in step (c),
(e) adding sodium borohydride solution to the solution obtained in step (d) to produce a dextran-guanidine-bisphosphonate conjugate,
(f) purifying the dextran-guanidine-bisphosphonate conjugate obtained in step (e), preferably purification is carried out by tangential flow filtration, to produce a purified dextran-guanidine-bisphosphonate conjugate,
(g) diluting the purified dextran-guanidine-bisphosphonate conjugate obtained in step (f) with sodium acetate and sodium chloride to produce a diluted dextran-guanidine-bisphosphonate conjugate, adjusting pH of the diluted dextran-guanidine-bisphosphonate conjugate to pH 6.0 - pH 7.0 and filtering the diluted dextran-guanidine-bisphosphonate conjugate.

2. The method according to claim 1, **characterized in that** the bisphosphonate is an aminobisphosphonate, preferably an alendronate group.

3. The method according to claim 1 or 2, **characterized in that** in step (a) pH is adjusted to pH 1.5 - pH 1.9, preferably to pH 1.55 - pH 1.8, more preferably to pH 1.6 - pH 1.7.

4. The method according to any one of the preceding claims, **characterized in that** in step (b) pH is adjusted to pH 7 - pH 7.7, preferably to pH 7.3 - pH 7.7, more preferably to pH 7.3 - pH 7.5.

5. The method according to any one of the preceding claims, **characterized in that** the step (a), step (c), or both step (a) and step (c), is conducted by stirring the solution in the dark.

6. The method according to any one of the preceding claims, **characterized in that** the step (d), step (e), or both step (d) and step (e) is conducted by stirring the solution at room temperature.

7. The method according to any one of the preceding claims, **characterized in that** the dextran-guanidine-bisphosphonate conjugate in step (g) is filtered through bacteria retentive filters.

## Patentansprüche

1. Verfahren zur Synthese eines Dextran-Guanidin-Bisphosphonat-Konjugats, **gekennzeichnet dadurch, dass** das Verfahren ein großtechnischer Eintopfprozess ist und das Verfahren die Abfolge der folgenden Schritte umfasst:
(a) Zugeben von Periodatsalz zu einer wässrigen Lösung, die Dextran umfasst, gefolgt von der Zugabe von Schwefelsäure,
(b) Zugeben von Natriumdihydrogenphosphat zu der in Schritt (a) erhaltenen Lösung, gefolgt von der Zugabe von Natriumhydroxid,
(c) Zugeben von Alendronsäure zu der in Schritt (b) erhaltenen Lösung,
(d) Zugeben von Aminoguanidin zu der in Schritt (c) erhaltenen Lösung,
(e) Zugeben von Natriumborhydrid-Lösung zu der in Schritt (d) erhaltenen Lösung, um ein Dextran-Guanidin-Bisphosphonat-Konjugat zu erzeugen,
(f) Reinigen des in Schritt (e) erhaltenen Dextran-Guanidin-Bisphosphonat-Konjugats, wobei die Reinigung vorzugsweise durch Tangentialflussfiltration ausgeführt wird, um ein gereinigtes Dextran-Guanidin-Bisphosphonat-Konjugat zu erzeugen,
(g) Verdünnen des in Schritt (f) erhaltenen gereinigten Dextran-Guanidin-Bisphosphonat-Konjugats mit Natriumacetat und Natriumchlorid, um ein verdünntes Dextran-Guanidin-Bisphosphonat-Konjugat zu erzeugen, Einstellen des pH-Wertes des verdünnten Dextran-Guanidin-Bisphosphonat-Konjugats auf pH 6,0 - pH 7,0 und Filtrieren des verdünnten Dextran-Guanidin-Bisphosphonat-Konjugats.

2. Verfahren nach Anspruch 1, **gekennzeichnet dadurch, dass** das Bisphosphonat ein Aminobisphosphonat, vorzugsweise eine Alendronatgruppe, ist.

3. Verfahren nach Anspruch 1 oder 2, **gekennzeichnet dadurch, dass** in Schritt (a) der pH-Wert auf pH 1,5 - pH 1,9, bevorzugt auf pH 1,55 - pH 1,8, bevorzugter auf pH 1,6 - pH 1,7, eingestellt wird.

4. Verfahren nach einem der vorhergehenden Ansprüche, **gekennzeichnet dadurch, dass** in Schritt (b) der pH-Wert auf pH 7 - pH 7,7, bevorzugt auf pH 7,3 - pH 7,7, bevorzugter auf pH 7,3 - pH 7,5, eingestellt wird.

5. Verfahren nach einem der vorhergehenden Ansprüche, **gekennzeichnet dadurch, dass** Schritt (a), Schritt (c), oder sowohl Schritt (a) als auch Schritt (c), ausgeführt wird, indem die Lösung im Dunkeln gerührt wird.

6. Verfahren nach einem der vorhergehenden Ansprüche, **gekennzeichnet dadurch, dass** Schritt (d), Schritt (e), oder sowohl Schritt (d) als auch Schritt (e), ausgeführt wird, indem die Lösung bei Raumtemperatur gerührt wird.

7. Verfahren nach einem der vorhergehenden Ansprüche, **gekennzeichnet dadurch, dass** das Dextran-Guanidin-Bisphosphonat-Konjugat in Schritt (g) durch Bakterienrückhaltefilter filtriert wird.

## Revendications

1. Procédé de synthèse d'un conjugué de dextrane-guanidine-bisphosphonate, **caractérisé en ce que** le procédé est un processus monotope à grande échelle et le procédé comprend la séquence des étapes consistant à :
(a) ajouter un sel de périodate à une solution aqueuse comprenant du dextrane, puis ajouter de l'acide sulfurique,
(b) ajouter du dihydrogénophosphate de sodium à la solution obtenue dans l'étape (a), puis ajouter de l'hydroxyde de sodium,
(c) ajouter de l'acide alendronique à la solution obtenue dans l'étape (b),
(d) ajouter de l'aminoguanidine à la solution obtenue dans l'étape (c),
(e) ajouter de la solution de borohydrure de sodium à la solution obtenue dans l'étape (d) pour produire un conjugué de dextrane-guanidine-bisphosphonate,
(f) purifier le conjugué de dextrane-guanidine-bisphosphonate obtenu dans l'étape (e), ladite purification étant effectuée préférablement par une filtration à flux tangentiel, pour produire un conjugué de dextrane-guanidine-bisphosphonate purifié,
(g) diluer le conjugué de dextrane-guanidine-bisphosphonate purifié obtenu dans l'étape (f) avec de l'acétate de sodium et du chlorure de sodium pour produire un conjugué de dextrane-guanidine-bisphosphonate dilué, ajuster la valeur pH du conjugué de dextrane-guanidine-bisphosphonate dilué à pH 6,0 - pH 7,0 et filtrer le conjugué de dextrane-guanidine-bisphosphonate dilué.

2. Procédé selon la revendication 1, **caractérisé en ce que** le bisphosphonate est un aminobisphosphonate, préférablement un groupe alendronate.

3. Procédé selon la revendication 1 ou 2, **caractérisé en ce que**, dans l'étape (a), le pH est ajusté à pH 1,5 - pH 1,9, préférablement à pH 1,55 - pH 1,8, plus préférablement à pH 1,6 - pH 1,7.

4. Procédé selon l'une des revendications précédentes, **caractérisé en ce que**, dans l'étape (b), le pH est ajusté à pH 7 - pH 7,7, préférablement à pH 7,3 - pH 7,7, plus préférablement à pH 7,3 - pH 7,5.

5. Procédé selon l'une des revendications précédentes, **caractérisé en ce que** l'étape (a), l'étape (c), ou les deux étapes (a) et (c), est réalisée par agitation de la solution dans l'obscurité.

6. Procédé selon l'une des revendications précédentes, **caractérisé en ce que** l'étape (d), l'étape (e), ou les deux étapes (d) et (e), est réalisée par agitation de la solution à température ambiante.

7. Procédé selon l'une des revendications précédentes, **caractérisé en ce que** le conjugué de dextrane-guanidine-bisphosphonate dans l'étape (g) est filtré à travers des filtres de rétention bactérienne.
